# EUROPEAN PATENT SPECIFICATION

(11) **EP 1 887 074 B1**
(45) Date of publication and mention of the grant of the patent: **14.09.2011**
(21) Application number: 07111678.4
(22) Date of filing: 03.07.2007
(51) Int. Cl.: C10L 1/238, C10L 1/226, C10L 10/18, C10L 10/02, F02M 61/18, F02M 65/00, F02B 77/04, C08F 8/30, C07C 241/04

(54) **Method and use for the prevention of fuel injector deposits**
Verfahren und Anwendung zum Vermeiden von Ablagerungen in einem Kraftstoffinjektor
Procédé et utilisation pour la prévention de dépôts d'injecteur de carburant

(30) Priority: 04.08.2006 EP 06118488
(43) Date of publication of application: 13.02.2008
(60) Divisional application: 09160925.5
(73) Proprietor: Infineum International Limited, Abingdon Oxfordshire OX13 6BB (GB)
(72) Inventor: Brekspear, Angela Priscilla, Dr., Abingdon Oxfordshire, OX13 6BB (GB); Caprotti, Rinaldo, Abingdon Oxfordshire, OX13 6BB (GB); Thomson, Russell Martin, Abingdon Oxfordshire, OX13 6BB (GB)
(74) Representative: Capaldi, Michael Joseph

(56) References cited:
- EP-A1- 0 632 123
- EP-A2- 1 312 796
- AU-B2- 413 800
- DE-A1- 10 318 135
- FR-A- 2 352 957
- FR-A- 2 876 750
- GB-A- 1 124 496
- GB-A- 1 365 081
- US-A- 2 395 642
- US-A- 3 375 092
- US-A1- 2004 237 929
- GRAUPNER O ET AL: "INJECTOR DEPOSIT TEST FOR MODERN DIESEL ENGINES" 5TH INTERNATIONAL COLLOQUIUM, 12 January 2005 (2005-01-12), pages 157-162, XP008073687 Technische Akademie Esslingen
- FEUER H ET AL: "THE REACTIONS OF SUCCINIC ANHYDRIDE WITH HYDRAZINE HYDRATE" JOURNAL OF THE AMERICAN CHEMICAL SOCIETY, AMERICAN CHEMICAL SOCIETY, WASHINGTON, DC, US, vol. 73, 1951, pages 4716-4719, XP001121824 ISSN: 0002-7863

## Description

This invention relates to a method for the removal or prevention of fuel injector deposits in diesel engines, in particular to the removal or prevention of fuel injector deposits in modem diesel engines. Uses of reaction products to remove or prevent fuel injector deposits and processes for the production of diesel fuel detergents are described.

There is continued legislative pressure to reduce emissions from diesel engines. In Europe by 2008, all new diesel engines must comply with the Euro V specification. This has resulted in the development of advanced fuel injection equipment characterised by fuel injectors which have complex spray-hole geometries, multiple and narrow spray-holes and which operate with high temperatures and pressures at the injector tips. As a consequence of this increasing severity in operating conditions, the injectors of modem common-rail diesel engines are prone to the formation of deposits. These deposits, which are found both inside and outside the spray-holes of the injector nozzles, contribute directly to loss in engine power and increase in smoke production.

The formation of deposits on diesel fuel injectors is not a new phenomenon and historically any problem has been adequately addressed by the use of conventional diesel detergent additives. It has been observed however, that the types of deposits formed under the more severe operating conditions of engines which are being developed to be Euro V compliant are not adequately removed or prevented by conventional diesel detergent additives. Although not wishing to be bound by any theory, it is presently thought that the formation of injector deposits in modem engines is exacerbated by the presence of minor amounts of metal-containing species in the fuel. Indeed, the Applicant's studies have indicated that the use of fuels with negligible amounts of metal-containing contamination do not result in any significant problems with deposits. However, normal diesel fuels will often contain low but measurable amounts of metal-containing contamination, for example, zinc, copper, iron and lead, and metal-containing species may also be deliberately added to perform other functions. Analysis of the deposits formed in modem diesel engines indicates that, in addition to the expected carbonaceous materials, metals such as zinc and copper can be detected. The present invention specifically addresses the removal and prevention of these new types of injector deposits.

US 3,375,092 discloses the product of the reaction between an alkyl succinic acid or anhydride in which the alkyl radical has from 8 to 24 carbon with a hydrazine. This product is said to be useful as an anti-icing additive for gasoline.

US 2,640,005 discloses succinhydrazides which are formed for example, by the reaction of hydrazine or hydrazine hydrate with the anhydride of a substituted succinic acid. These species are taught as having utility as fungicides.

US 3,723,460 discloses that the species formed by the reaction of e.g. polyisobutenyl- substituted succinic acid or anhydride with hydrazine can be used as fuel and motor oil additives. The intermediate reaction products are preferably post-reacted with further compounds for example, those with displaceable halogens, alkylene oxides etc., but may also be used alone. The species are discussed as being of sufficient detergent strength to clean and maintain clean, a gasoline engine induction system but not of sufficient detergent strength to promote the formation of gasoline-in-water emulsions. They are also able to function as a carburettor cleaner. There is no disclosure of use in diesel engine systems.

WO 2004/029183 discloses ashless anti-wear, anti-fatigue and extreme pressure additives for lubricating oils. These have the formula: 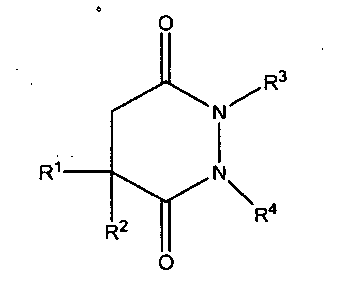 where group R¹ may be e.g. alkyl and groups R²⁻⁴ may be hydrogen or similar to R¹. The additives are prepared by reacting e.g. an alkyl succinic anhydride with hydrazine hydrate.

EP 0 632 123 A1 relates to diesel fuel compositions containing nitrogen-containing dispersants. The dispersant may be chosen from a very wide range of possible species, including those derived from hydrazines. The dispersants are characterised in that the numerical value obtained by multiplying the percentage of nitrogen in the dispersant by the weight average molecular weight of the dispersant is between 45,000 and 100,000.

In accordance with a first aspect, the present invention provides a method of substantially removing, or reducing the occurrence of, injector deposits in a diesel engine operated using a diesel fuel containing between 0.1 and 50 ppm by weight, based on the weight of the diesel fuel of a metal-containing species, expressed in terms of the total weight of metal in the species the method comprising adding to the diesel fuel the reaction product between a hydrocarbyl-substituted succinic acid or anhydride and hydrazine, wherein the diesel engine is equipped with fuel injectors having a plurality of spray-holes, each spray-hole having an inlet and an outlet, and wherein the fuel injectors have one or more of the following characteristics:
(i) spray-holes which are tapered such that the inlet diameter of the spray-holes is greater than the outlet diameter;
(ii) spray-holes having an outlet diameter of 0.10 mm or less;
(iii) spray-holes where an inner edge of the inlet is rounded;
(iv) 6 or more spray-holes;
(v) an operating tip temperature in excess of 250°C.

In accordance with a second aspect, the present invention provides the use of the reaction product between a hydrocarbyl substituted succinic acid or anhydride and hydrazine as an additive in a diesel fuel to substantially remove, or reduce the occurrence of, injector deposits in a diesel engine, the diesel engine being equipped with fuel injectors having one or more of characteristics (i) to (v) as defined in relation to the first aspect and operated using a diesel fuel containing between 0.1 and 50 ppm by weight, based on the weight of the diesel fuel of a metal-containing species, expressed in terms of the total weight of metal in the species.

It has been found that the reaction products used in the first and second aspects are particularly effective at reducing the incidence of deposits in modem diesel engine fuel injectors, and much more effective than the widely used PIBSA-PAM detergents under similar conditions. It was surprising to note however that in older type diesel engines, such as those used in the industry standard XUD-9 detergency test, the reaction products of use in the present invention were outperformed by conventional PIBSA-PAM detergents.

As discussed above, the incidence of injector deposits appears to be connected to the presence of metal-containing species in the fuel. Some diesel fuels will contain no measurable metal content, in which case the incidence of injector deposits will be reduced. However, the presence or absence of metal-containing species in diesel fuels is generally not apparent to the user and will vary with fuel production, even with fuels from the same supplier. The present invention is thus useful in those instances where metal-containing species are present and also as a preventative measure to lessen the impact of injector deposits when re-fuelling with a fuel of unknown metal content.

In the context of all aspects of the present invention, substantial removal of injector deposits should be taken to mean that deposits which may be present on the inside or outside of the spray-holes of the injector nozzles are removed to the extent that the proper functioning of the injector is not significantly impaired. This may be determined for example by measuring increases in exhaust smoke or loss in engine torque. It is not required that all traces of injector deposit are removed. Similarly, a reduction in the occurrence of injector deposits does not require that no deposits whatsoever are formed, only again that the amount of any deposit which may form is not sufficient to significantly impair the proper functioning of the injector.

It is presently thought that the characteristics (i) to (v) of the fuel injectors all contribute to the formation of injector deposits. It has been observed that diesel engines employing fuel injectors which have a plurality of these characteristics are more prone to deposit formation. Thus in embodiments of the invention, the fuel injectors have two, preferably three, more preferably four, most preferably all five of characteristics (i) to (v).

In a preferred embodiment, the fuel injectors have at least characteristics (i) and (ii). In a more preferred embodiment, the fuel injectors have at least characteristics (i), (ii) and (iii). In an even more preferred embodiment, the fuel injectors have at least characteristics (i), (ii), (iii) and (iv).

The reaction between the hydrocarbyl-substituted succinic acid or anhydride and hydrazine produces a mixture of reaction products (as discussed hereinbelow). This mixture is made up from species which have a range of molecular weights. These range from low molecular weight species, being composed of one moiety of hydrocarbyl-substituted succinic acid or anhydride and one or two moieties of hydrazine, to species composed of more than one moiety of hydrocarbyl-substituted succinic acid or anhydride and one or more moieties of hydrazine. These latter species have relatively higher molecular weights than the former. It has been observed that most effective detergency is obtained by employing a reaction product which contains a significant proportion of higher molecular weight species. Accordingly, it is advantageous that at least 25%, preferably at least 50%, most preferably at least 80% by weight of the reaction product between the hydrocarbyl-substituted succinic acid or anhydride and hydrazine has a molecular weight which is more than 2 times, preferably more than 2.5 times, the average molecular weight of the hydrocarbyl group of the hydrocarbyl-substituted succinic acid or anhydride.

Expressed in terms of EP 0 632 123 A1 discussed above, preferably, the reaction product of the present invention is such that the numerical value obtained by multiplying the percentage of nitrogen in the product by the weight average molecular weight of the product is in excess of 105,000, more preferably in excess of 110,000, for example between 110,000 and 250,000.

The various features of the invention, which are applicable to all aspects will now be described in more detail.

### (a) The reaction product

This comprises the product of the reaction between a hydrocarbyl-substituted succinic acid or anhydride and hydrazine.

### (i) Hydrocarbyl-substituted succinic acid or anhydride.

As used in this specification the term "hydrocarbyl" refers to a group having a carbon atom directly attached to the rest of the molecule and having a hydrocarbon or predominantly hydrocarbon character. They may be saturated or unsaturated, linear or branched. Preferably, the hydrocarbyl groups are hydrocarbon groups. These groups may contain non-hydrocarbon substituents provided their presence does not alter the predominantly hydrocarbon character of the group. Examples include keto, halo, nitro, cyano, alkoxy and acyl. The groups may also or alternatively contain atoms other than carbon in a chain otherwise composed of carbon atoms. Suitable hetero atoms include, for example, nitrogen, sulphur, and oxygen. Advantageously, the hydrocarbyl groups are alkyl groups.

Preferably, the hydrocarbyl group of the hydrocarbyl-substituted succinic acid or anhydride comprises a C₈ - C₃₆ group, preferably a C₈ - C₁₈ group, preferably a C₈-C₁₆ group. Non-limiting examples include dodecyl, hexadecyl and octadecyl. Alternatively, the hydrocarbyl group may be a polyisobutylene group with a number average molecular weight of between 200 and 2500, 400 and 2500, preferably between 800 and 1200. Mixtures of species with different length hydrocarbyl groups are also suitable, e.g. a mixture of C₁₆- C₁₈ groups.

The hydrocarbyl group is attached to a succinic acid or anhydride moiety using methods known in the art. Additionally, or alternatively, suitable hydrocarbyl-substituted succinic acids or anhydrides are commercially available e.g. dodecylsuccinic anhydride (DDSA), hexadecylsuccinic anhydride (HDSA), octadecylsuccinic anhydride (ODSA) and polyisobutylsuccinic anhydride (PIBSA).

### (ii) Hydrazine

Hydrazine has the formula:

NH₂-NH₂

Hydrazine may be hydrated or non-hydrated. Hydrazine monohydrate is preferred.

### (iii) Reaction of (i) and (ii)

The reaction between the hydrocarbyl-substituted succinic acid or anhydride and hydrazine produces a variety of products. As noted above, it is preferable for good detergency that the reaction product contains a significant proportion of species with relatively high molecular weight. The precise nature of the species produced in the reaction has not yet been fully elucidated however, it is presently thought that a major high molecular weight product of the reaction is an oligomeric species predominantly of the structure: 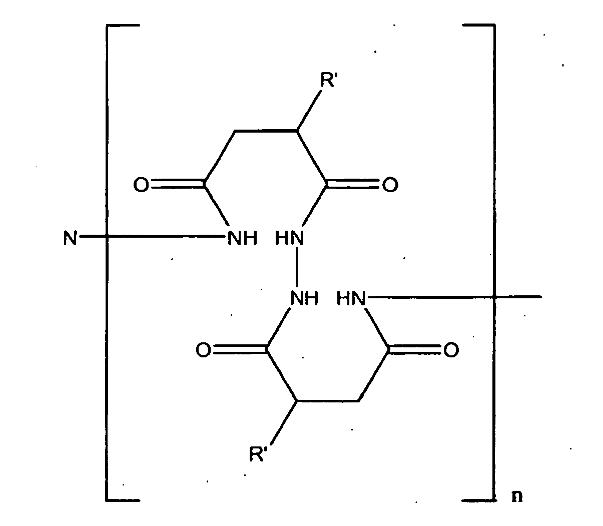 where n is an integer and greater than 1, preferably between 2 and 10, more preferably between 2 and 7, for example 3, 4 or 5. Each end of the oligomer may be capped by one or more of a variety of groups. Some possible examples of these terminal groups include:

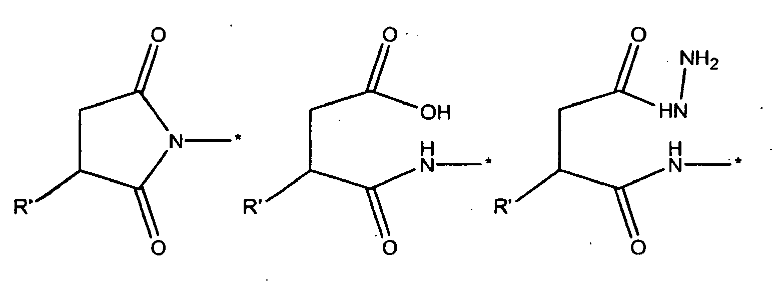

Alternatively, the oligomeric species may form a ring having no terminal groups:

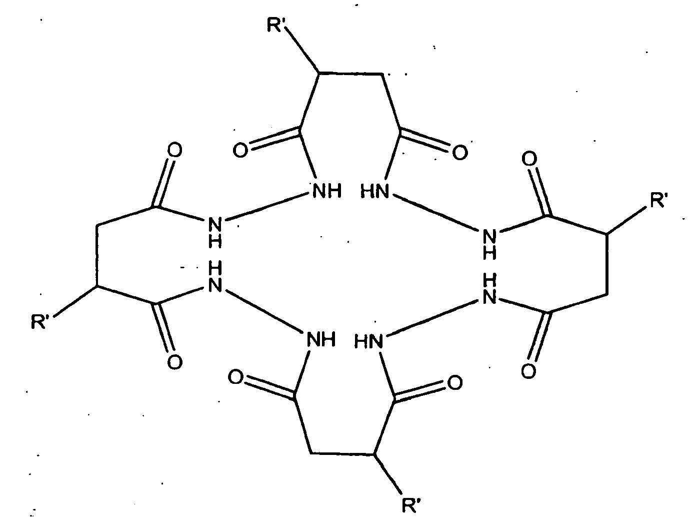

Also thought to be present is a species of the structure: 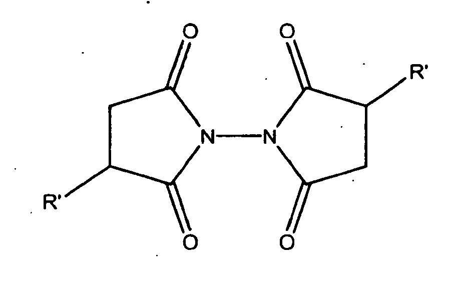 where R' represents the hydrocarbyl substituent. It should be noted that it is also within the scope of the present invention to use more than one hydrocarbyl-substituted succinic acid or anhydride in which case the groups R' in the above structures may be different from one another.

Both of the above structures contain at least two moieties derived from the hydrocarbyl-substituted succinic acid or anhydride. The molecular weights of these species are thus more than twice the average molecular weight of the hydrocarbyl substituent R'. In the context of the present invention the species are thus of relatively high molecular weight.

As lower molecular weight reaction products, species of the following structures are also thought to be present:

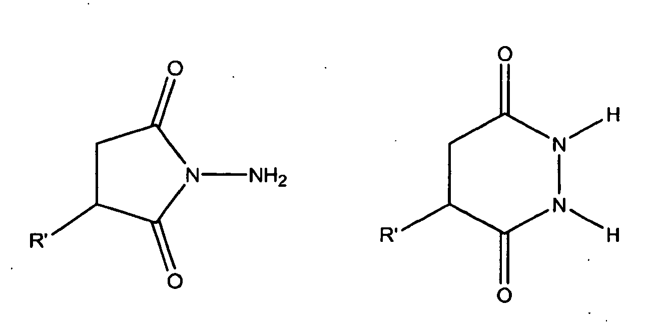

Further possible minor products include:

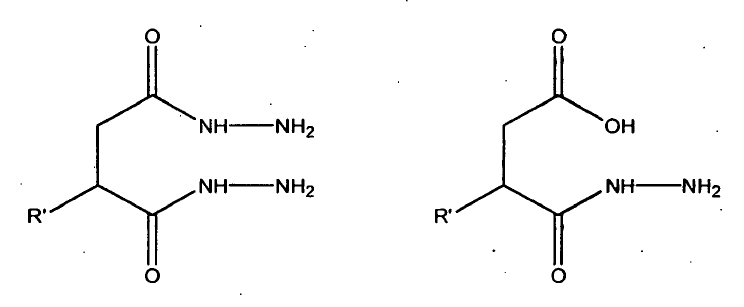

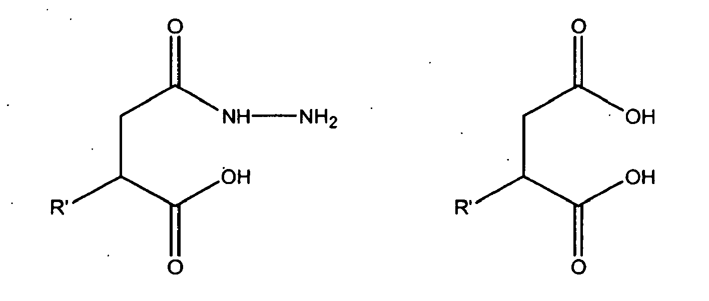

There may also be some salt formation resulting in species of the following structures:

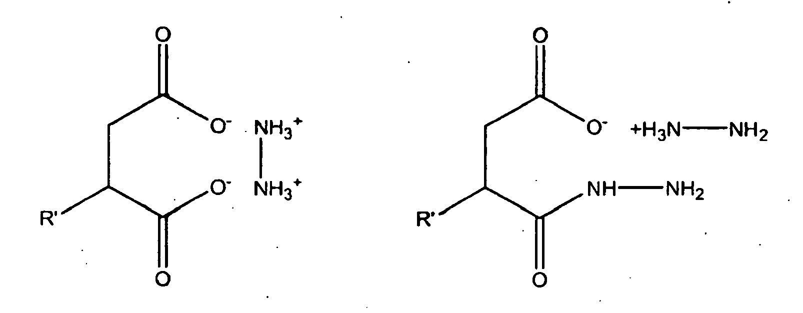

The general synthesis of the reaction products used in the present invention has been described in the art, for example, US 3,375,092, US 2,640,005 and US 3,723,460 cited hereinabove. A range of possible reaction schemes and products has also been given by Feuer et al., in Jn. Amer. Chem. Soc, 73 (1951) pp.4716-4719*.* By way of example a possible preparative route is as follows.

A charge of alkyl-substituted succinic anhydride together with an equal weight of solvent, e.g. toluene is heated to ca. 50°C under nitrogen. The desired amount of hydrazine hydrate is added drop-wise causing an exotherm. Once addition is complete, the reaction mixture is heated to reflux for several hours. The mixture is then water/solvent stripped and the temperature raised to 180°C under reduced pressure.

Preferably, the hydrocarbyl-substituted succinic acid or anhydride and hydrazine are reacted in a molar ratio of between 2:1 and 1:4, more preferably between 1:1 - 1:3.

Preferably, the reaction product between the hydrocarbyl-substituted succinic acid or anhydride and hydrazine is added to the diesel fuel in an amount of between 10 and 500 ppm by weight, based on the weight of the fuel, more preferably between 20 and 100ppm.

### (b) The diesel fuel

Preferably, the diesel fuel is a petroleum-based fuel oil, especially a middle distillate fuel oil. Such distillate fuel oils generally boil within the range of from 110°C to 500°C, e.g. 150°C to 400°C. The fuel oil may comprise atmospheric distillate or vacuum distillate, cracked gas oil, or a blend in any proportion of straight run and thermally and/or refinery streams such as catalytically cracked and hydro-cracked distillates.

Other examples of diesel fuels include Fischer-Tropsch fuels. Fischer-Tropsch fuels, also known as FT fuels, include those described as gas-to-liquid (GTL) fuels, biomass-to-liquid (BTL) fuels and coal conversion fuels. To make such fuels, syngas (CO + H₂) is first generated and then converted to normal paraffins by a Fischer-Tropsch process. The normal paraffins may then be modified by processes such as catalytic cracking/reforming or isomerisation, hydrocracking and hydroisomerisation to yield a variety of hydrocarbons such as iso-paraffins, cyclo-paraffins and aromatic compounds. The resulting FT fuel can be used as such or in combination with other fuel components and fuel types. Also suitable are diesel fuels derived from plant or animal sources such as FAME. These may be used alone or in combination with other types of fuel.

Preferably, the diesel fuel has a sulphur content of at most 0.05% by weight, more preferably of at most 0.035% by weight, especially of at most 0.015%. Fuels with even lower levels of sulphur are also suitable such as, fuels with less than 50ppm sulphur by weight, preferably less than 20 ppm, for example 10ppm or less.

As discussed herein, the Applicants have observed that the problems associated with the formation of injector deposits in engines being developed to be Euro V compliant are associated with the presence of metal-containing species in the diesel fuel. Commonly when present, metal-containing species will be present as a contaminant, for example through the corrosion of metal and metal oxide surfaces by acidic species present in the fuel. In use, fuels such as diesel fuels routinely come into contact with metal surfaces for example, in vehicle fuelling systems, fuel tanks, fuel transportation means etc. Typically, metal-containing contamination will comprise metals such as zinc, iron, copper and lead.

In addition to metal-containing contamination which may present in diesel fuels there are circumstances where metal-containing species may deliberately be added to the fuel. For example, as is known in the art, metal-containing fuel-borne catalyst species may be added to aid with the regeneration of particulate traps. Such catalysts are often based on metals such as iron, cerium, Group I and Group II metals e.g., calcium and strontium, either as mixtures or alone. Also used are platinum and manganese. The presence of such catalysts may also give rise to injector deposits when the fuels are used in engines being developed to be Euro V compliant.

Metal-containing contamination, depending on its source, may be in the form of insoluble particulates or soluble compounds or complexes. Metal-containing fuel-borne catalysts are often soluble compounds or complexes or colloidal species. It will be understood that metal-containing species in the context of the present invention include both species which are metallic and those where the metal constituent is in compounded form.

In an embodiment, the metal-containing species comprises a fuel-borne catalyst.

In a preferred embodiment, the metal-containing species comprises zinc.

Typically, the amount of metal-containing species in the diesel fuel, expressed in terms of the total weight of metal in the species, is between 0.1 and 50 ppm by weight, for example between 0.1 and 10 ppm by weight, based on the weight of the diesel fuel.

### (c) Fuel injector characteristics

Historically, diesel engine fuel injectors have been simple in design. In recent years, the connection between injector design and engine performance has become better understood. For example, the knowledge that a fine distribution of fuel droplets promotes a decrease in emissions has led to a gradual narrowing of fuel injector spray-holes and increased injector pressures. As mentioned hereinabove, the drive to meet the upcoming Euro V emissions specification has led to further advances in fuel injector design.

### (i) Tapered spray-holes

The majority of fuel injectors have spray-holes which are uniform in cross-section. In the present invention, preferably the spray-holes are tapered such that diameter at the point where the fuel enters the spray-hole (the inlet) is greater than the diameter at the point where the fuel exits the spray-hole (the outlet). Most typically, the spray-holes will be conical or frusto-conical in shape.

### (ii) Spray-hole diameter

The spray-holes preferably have an outlet diameter of 0.10mm or less, more preferably 0.08mm or less. This may be compared to injectors of 10 to 15 years ago which had spray-holes of typically 0.25mm.

### (iii) Rounded spray-holes

In the context of the present invention, rounded spray-holes are those where the inner edge of the inlet of the hole has been formed, smoothed or eroded to have a curved or radial profile, rather than an angled profile.

### (iv) Multiple spray-holes

Historically, fuel injectors have had up to four spray-holes. The present invention relates to fuel injectors preferably having 6 or more spray-holes, for example 6, 7, 8, 9, 10 or more. It is anticipated that future designs of fuel injectors will have even more spray-holes.

### (v) Operating tip temperature

The combination of lower fuel flow due to a large number of spray-holes, higher fuel pressures and complex spray-hole geometry leads to increased injector tip temperatures. Typically, the fuel injectors will have an operating tip temperature in excess of 250°C, preferably in excess of 300°C. It will be understood that the operating tip temperature of the fuel injectors refers to the temperature of the injector tip during normal running of the diesel engine. Those skilled in the art will be aware of methodologies to measure the injector tip temperature, for example by the use of suitably placed thermocouples.

Characteristics (i) to (iv) result in a less turbulent fuel flow through the injector. Whilst this is generally advantageous, it lessens the possibility for the fuel to physically erode any deposits which may be present. The increase in operating tip temperature is also thought to contribute to the formation of deposits.

It has also been observed that the reaction products which are the subject of the present invention are effective to improve the lubricity of low sulphur-content diesel fuels. The reaction product of dodecyl-substituted succinic anhydride and hydrazine was found to be particularly effective in this regard.

The invention will now be described by way of example only.

### Preparative routes

### Example 1

Dodecylsuccinic anhydride (200g, 0.75 mol) was weighed into a 11, three neck, roundbottom flask together with toluene (200g). Under nitrogen and with stirring, the temperature was raised to ca. 50°C and hydrazine monohydrate (37.59g, 0.75 mol) added dropwise. Once addition was complete, the mixture was heated to reflux for 5 hours. Toluene was removed at 40°C until no more bubbling was seen and then the product was held for 1 hour at 0 mbar and 40°C.

The product produced in the reaction contained around 10% by weight of species having a molecular weight more than 2 times the molecular weight of the hydrocarbyl group of the dodecylsuccinic anhydride reactant.

### Example 2

Dodecylsuccinic anhydride (200g, 0.75 mol) was weighed into a 11, three neck, roundbottom flask together with toluene (200g). Under nitrogen and with stirring, the temperature was raised to ca. 50°C and hydrazine monohydrate (112.76g, 2.25 mol) added dropwise. Once addition was complete, the mixture was heated to reflux for 5 hours. Toluene was removed at 40°C until no more bubbling was seen and then the product was held for 4 hours at 0 mbar and 180°C.

The product produced in the reaction contained around 70% by weight of species having a molecular weight more than 2 times the molecular weight of the hydrocarbyl group of the dodecylsuccinic anhydride reactant.

### Example 3

A further example using dodecylsuccinic anhydride in a synthesis similar to Example 2 produced a product containing around 84% by weight of species having a molecular weight more than 2 times the molecular weight of the hydrocarbyl group of the dodecylsuccinic anhydride reactant.

### Effect of process variables

Example 2 was repeated varying the temperature and pressure of the final stage following the removal of toluene. Table 1 below shows the effect of these variables on the molecular weight distribution of the products obtained. In the Table, high MW species are those having a molecular weight more than 2 times the molecular weight of the hydrocarbyl group of the dodecylsuccinic anhydride reactant.

**Table 1**

| **Temperature / °C** | **Pressure / mbar** | **% of high MW species** |
|---|---|---|
| 40 | 35 | 20 |
| 60 | 35 | 15 |
| 80 | 35 | 17 |
| 100 | 35 | 18 |
| 120 | 35 | 25 |
| 140 | 35 | 33 |
| 160 | 35 | 46 |
| 180 | 35 | 62 |
| 180 | 0 | 76 |

Following the routes of Examples 1 and 2, further species were prepared by reacting hydrazine mono-hydrate with a C₂₄-alkyl succinic anhydride and a polyisobutylene-substituted succinic anhydride (mol weight of PIB ca. 1000).

### Test protocol

The protocol used is described by Graupner et al. "Injector deposit test for modern diesel engines ", Technische Akademie Esslingen, 5th International Colloquium, 12-13 Jan 2005, 3.10, p157, Edited by Wilfried J Bartz*.* Briefly, the protocol aims to replicate the operating conditions in a modem diesel engine with an emphasis on the fuel injector tip. The test is split into five stages:
a) an iso-speed measurement of engine power output
b) an 8 hour endurance run
c) an extended soaking period (3 to 8 hours) during which the engine is stopped and allowed to cool
d) a second 8 hour endurance run
e) an iso-speed measurement of engine power output.

For the data presented herein, the five stages above were used however, stages b) ,c) and d) can be repeated any number of times to suit the testing programme being undertaken. Also, stages a) and e) may be omitted but are useful to improve understanding of the results. Results are reported as the difference between the average torque at the start of the test during stage a) and the average torque at the end of the test during stage e). Alternatively, if the isospeed procedure is not run, the measured difference between starting torque at full load/full speed and final load/speed can be used. Differences in smoke production are also noted. The formation of injector deposits will have a negative influence on the final power output and will increase the amount of smoke observed. The injectors used had the physical characteristics (i) - (v) described above.

To replicate the conditions expected in a modem diesel engine, a small amount of metal contamination in the form of zinc neodecanoate was added to the fuel used to run the engine.

The fuel used was a low-sulphur content diesel fuel with the characteristics shown in Table 2 below.

**Table 2**

| **Test description** | **Value** | **Units** |
|---|---|---|
| sulphur content | 0.0005 | mass % |
| cetane number | 55.4 | - |
| density @ 15°C | 844.9 | kgm⁻³ |
| distillation characteristics | | |
| D5% | 204.8 | °C |
| D10% | 211.6 | °C |
| D20% | 222.2 | °C |
| D30% | 232.2 | °C |
| D40% | 242.1 | °C |
| D50% | 252.3 | °C |
| D60% | 262.8 | °C |
| D70% | 275.1 | °C |
| D80% | 290.5 | °C |
| D90% | 315.1 | °C |
| D95% | 337.1 | °C |
| FBP | 353.6 | °C |
| IBP | 179.7 | °C |
| kinematic viscosity @ 20°C | 3.935 | cSt |
| kinematic viscosity @ 40°C- D445 | | |
| cloud point | -14.0 | °C |
| CFPP | -33.0 | °C |

For comparative purposes, the species of the invention were tested in the industry standard XUD9 detergency test. A commercial PIBSA-PAM detergent was tested also. The results are given in Table 3 below.

**Table 3**

| **Species** | **Treat rate wppm (active ingredient)** | **Needle lift in XUD9** |
|---|---|---|
| Untreated fuel | - | 92 |
| PIBSA-PAM | 30 | 57 |
| PIBSA-PAM | 60 | 53 |
| PIBSA-PAM | 100 | 8 |
| PIBSA-PAM | 279 | 14 |
| Example 1 | 60 | 83 |
| Example 2 | 60 | 83 |
| Example 3 | 60 | 93 |
| PIB 1000 hydrazide | 60 | 77 |
| C₂₄-SA hydrazide | 60 | 78 |
| Example 2 | 300 | 83 |
| PIB1000 hydrazide | 300 | 76 |

These results show that the commercial PIBSA-PAM detergent gave the expected excellent performance in the XUD-9 test. Contrastingly, the hydrazine species performed poorly, even at high treat rates.

The species were then tested using the test protocol described above. Results are given in Table 4 below. 3 ppm of Zn in the form of zinc neodecanoate was added to the fuel for all tests (except for the untreated fuel alone).

**Table 4**

| **Species** | **Treat rate wppm (active ingredient)** | **Torque loss** |
|---|---|---|
| Untreated fuel | - | 4.3% |
| Untreated fuel + 3 ppm Zn | - | 17.2% |
| PIBSA-PAM | 60 | 13.7% |
| PIB1000 hydrazide | 60 | 9.7% |
| C₂₄-SA hydrazide | 60 | 7.1 % |
| Example 1 | 60 | 12.0% |
| Example 2 | 60 | 5.2% |

Table 5 below shows a further result for the product of Example 3.

**Table 5**

| **Species** | **Treat rate wppm (active ingredient)** | **Torque loss** |
|---|---|---|
| Untreated fuel | - | 1.3% |
| Untreated fuel + 3 ppm Zn | - | 10.0% |
| PIBSA-PAM | 60 | 9.2% |
| Example 3 | 60 | 0.9% |

The results show that the addition of zinc to the untreated fuel gives rise to a large increase in torque loss. The commercial PIBSA-PAM detergent only gave a marginal improvement. All hydrazine species provided a greater improvement than the commercial detergent. Particularly good performance was obtained for the species of Example 2 and Example 3 which both contained a high proportion of the higher molecular weight species.

The results in Table 6 below also illustrate the effect of the molecular weight distribution of the species on torque loss. Again, all tests contained 3 ppm of Zn in the form of zinc neodecanoate. The species used were the products of the reaction between dodecylsuccinic anhydride and hydrazine mono-hydrate following the routes of Examples 1 and 2 above and were present in the fuel at 60 wppm.

**Table 6**

| **% of high MW species** | **Torque loss** |
|---|---|
| 90.0 | 0.1 % |
| 73.4 | 4.2% |
| 72.0 | 5.2% |
| 70.0 | 5.2% |
| 10.0 | 12.0% |

These results confirm the increased effectiveness of the materials which contain the highest percentage of higher molecular weight species.

## Claims

1. A method of substantially removing, or reducing the occurrence of, injector deposits in a diesel engine operated using a diesel fuel containing between 0.1 and 50ppm by weight, based on the weight of the diesel fuel of a metal-containing species, expressed in terms of the total weight of metal in the species, the method comprising adding to the diesel fuel the reaction product between a hydrocarbyl-substituted succinic acid or anhydride and hydrazine, wherein the diesel engine is equipped with fuel injectors having a plurality of spray-holes, each spray-hole having an inlet and an outlet, and herein the fuel injectors have one or more of the following characteristics:
(i) spray-holes which are tapered such that the inlet diameter of the spray-holes is greater than the outlet diameter;
(ii) spray-holes having an outlet diameter of 0.10 mm or less;
(iii) spray-holes where an inner edge of the inlet is rounded;
(iv) 6 or more spray-holes;
(v) an operating tip temperature in excess of 250°C.

2. A method according to claim 1 wherein the fuel injectors have two, preferably three, more preferably four, most preferably all five of characteristics (i) to (v).

3. A method according to claim 1 or claim 2 wherein the fuel injectors have at least characteristics (i) and (ii), preferably at least characteristics (i), (ii) and (iii), more preferably at least characteristics (i), (ii), (iii) and (iv).

4. A method according to any preceding claim wherein at least 25%, preferably at least 50%, more preferably at least 30% by weight of the reaction product between the hydrocarbyl-substituted succinic acid or anhydride and hydrazine has a molecular weight which is more than 2 times, preferably more than 2.5 times, the average molecular weight of the hydrocarbyl group of the hydrocarbyl-substituted succinic acid or anhydride.

5. A method according to any preceding claim wherein the hydrocarbyl group of the hydrocarbyl-substituted succinic acid or anhydride comprises a C₈-C₃₆ group, preferably a C₈-C₁₆ group; or a polyisobutylene group with a number average molecular weight of between 400 and 2500, preferably between 800 and 1200.

6. A method according to any preceding claim wherein the hydrocarbyl-substituted succinic acid or anhydride and hydrazine are reacted in a molar ratio of 2:1-1:4, preferably, 1:1-1:3.

7. A method according to any preceding claim wherein the reaction product between the hydrocarbyl-substituted succinic acid or anhydride and hydrazine is added to the diesel fuel in an amount of between 10 and 500 ppm by weight, preferably between 20 and 100ppm based on the weight of the fuel.

8. A method according to any preceding claim wherein the metal-containing species comprises zinc, copper, iron, lead, cerium, a Group I or II metal, platinum or manganese.

9. A method according to claim 8 wherein the metal-containing species comprises zinc.

10. A method according to any preceding claim wherein the metal-containing species comprises a fuel-borne catalyst.

11. A method according to any preceding claim wherein the amount of metal-containing species in the diesel fuel, expressed in terms of the total weight of metal in the species, is between 1 and 50 ppm by weight, based on the weight of the diesel fuel.

12. The use of the reaction product between a hydrocarbyl substituted succinic acid or anhydride and hydrazine as an additive in a diesel fuel to substantially remote, or reduce the occurrence of, injector deposits in a diesel engine, the diesel engine being equipped with fuel injectors having one or more of characteristics (i) to (v) as defined in claim 1 and operated using a diesel fuel containing between 0.1 and 50ppm by weight, based on the weight of the diesel fuel of a metal-containing species, expressed in terms of the total weight of metal in the species.

## Patentansprüche

1. Verfahren zur im Wesentlichen vollständigen Entfernung oder zur Verminderung des Auftretens von Ablagerungen an Einspritzdüsen in einem Dieselmotor, der mit Dieselkraftstoff betrieben wird, der bezogen auf das Gewicht des Dieselkraftstoffs zwischen 0,1 und 50 Gew.-ppm an einer Metall enthaltenden Spezies enthält, ausgedrückt als das Gesamtgewicht an Metall in der Spezies, bei welchem Verfahren dem Dieselkraftstoff das Reaktionsprodukt zwischen mit Kohlenwasserstoff substituierter Bernsteinsäure oder mit Kohlenwasserstoff substituiertem Bernsteinsäureanhydrid und Hydrazin zugesetzt wird, wobei der Dieselmotor mit Einspritzdüsen ausgerüstet ist, die eine Vielzahl von Düsenbohrungen aufweisen, wobei jede Düsenbohrung einen Einlass und einen Auslass aufweist, und wobei die Einspritzdüsen eine oder mehrere der folgenden Eigenschaften aufweisen:
(i) Düsenbohrungen, die sich so verjüngen, dass der Einlassdurchmesser der Düsenbohrungen größer ist als der Auslassdurchmesser,
(ii) Düsenbohrungen mit einem Auslassdurchmesser von 0,10 mm oder weniger,
(iii) Düsenbohrungen, bei denen die innere Kante des Einlasses abgerundet ist,
(iv) 6 oder mehr Düsenbohrungen,
(v) eine Spitzenbetriebstemperatur von über 250°C.

2. Verfahren nach Anspruch 2, bei dem die Einspritzdüsen zwei, vorzugsweise drei, insbesondere vier, am meisten bevorzugt alle fünf Eigenschaften (i) bis (v) aufweisen.

3. Verfahren nach Anspruch 1 und Anspruch 2, bei dem die Einspritzdüsen mindestens die Eigenschaften (i) und (ii), vorzugsweise mindestens die Eigenschaften (i), (ii) und (iii), insbesondere mindestens die Eigenschaften (i), (ii), (iii) und (iv) aufweisen.

4. Verfahren nach einem der vorhergehenden Ansprüche, bei dem mindestens 25 Gew.-%, vorzugsweise mindestens 50 Gew.-% und insbesondere mindestens 80 Gew.-% des Reaktionsprodukts zwischen der mit Kohlenwasserstoff substituierten Bernsteinsäure oder des mit Kohlenwasserstoff substituierten Bernsteinsäureanhydrids und Hydrazin ein Molekulargewicht aufweisen, das mehr als das zweifache, vorzugsweise mehr als das 2,5-fache des durchschnittlichen Molekulargewichts der Kohlenwasserstoffgruppe der mit Kohlenwasserstoff substituierten Bernsteinsäure oder des mit Kohlenwasserstoff substituierten Bernsteinsäureanhydrids beträgt.

5. Verfahren nach einem der vorhergehenden Ansprüche, wobei die Kohlenwasserstoffgruppe der mit Kohlenwasserstoff substituierten Bernsteinsäure oder des mit Kohlenwasserstoff substituierten Bernsteinsäureanhydrids eine C₈- bis C₃₆-Gruppe, vorzugsweise eine C₈- bis C₁₆-Gruppe oder eine Polyisobutylengruppe mit einem Molekulargewicht (Zahlenmittel) von zwischen 400 und 2500, vorzugsweise zwischen 800 und 1200 umfasst.

6. Verfahren nach einem der vorhergehenden Ansprüche, bei dem die mit Kohlenwasserstoff substituierte Bernsteinsäure oder das mit Kohlenwasserstoff substituierte Bernsteinsäureanhydrid und Hydrazin in einem molaren Verhältnis von 2:1 bis 1:4, vorzugsweise 1:1 bis 1:3 umgesetzt werden.

7. Verfahren nach einem der vorhergehenden Ansprüche, bei dem das Reaktionsprodukt zwischen der mit Kohlenwasserstoff substituierten Bernsteinsäure oder des mit Kohlenwasserstoff substituierten Bernsteinsäureanhydrids und Hydrazin dem Dieselkraftstoff in einer Menge von zwischen 10 und 500 Gew.-ppm, vorzugsweise zwischen 20 und 100 ppm, bezogen auf das Gewicht des Treibstoffs, zugesetzt wird.

8. Verfahren nach einem der vorhergehenden Ansprüche, bei dem die Metall enthaltende Spezies Zink, Kupfer, Eisen, Blei, Cer, ein Metall aus Gruppe I oder II, Platin oder Mangan umfasst.

9. Verfahren nach Anspruch 8, bei dem die Metall enthaltende Spezies Zink umfasst.

10. Verfahren nach einem der vorhergehenden Ansprüche, bei dem die Metall enthaltende Spezies einen Kraftstoff-getragenen Katalysator (Fuel Born Catalyst) umfasst.

11. Verfahren nach einem der vorhergehenden Ansprüche, bei dem die Menge an Metall enthaltender Spezies in dem Dieselkraftstoff, ausgedrückt als das Gesamtgewicht an Metall in der Spezies, zwischen 1 und 50 Gew.-ppm beträgt, bezogen auf das Gewicht des Dieselkraftstoffs.

12. Verwendung des Reaktionsprodukts zwischen mit Kohlenwasserstoff substituierter Bernsteinsäure oder mit Kohlenwasserstoff substituiertem Bernsteinsäureanhydrid und Hydrazin als Additiv in einem Dieselkraftstoff, um Ablagerungen an Einspritzdüsen in einem Dieselmotor im Wesentlichen zu entfernen oder deren Auftritt zu vermindern, wobei der Dieselmotor mit Einspritzdüsen ausgestattet ist, die eine oder mehrere der Eigenschaften (i) bis (v) wie in Anspruch 1 definiert aufweisen, und der unter Verwendung eines Dieselkraftstoffs betrieben wird, der bezogen auf das Gewicht des Dieselkraftstoffs zwischen 0,1 und 50 Gew.-ppm an Metall enthaltender Spezies enthält, ausgedrückt als das Gesamtgewicht an Metall in der Spezies.

## Revendications

1. Procédé pour éliminer substantiellement les, ou réduire l'apparition de, dépôts dans les injecteurs dans un moteur diesel fonctionnant en utilisant un carburant diesel contenant 0,1 à 50 ppm en poids, sur la base du poids du carburant diesel, d'une entité contenant un métal, la quantité étant exprimée sur la base du poids total du métal dans l'entité, le procédé comprenant l'addition au carburant diesel du produit de réaction entre un acide ou anhydride succinique à substituant hydrocarbyle et de l'hydrazine, dans lequel le moteur diesel est équipé d'injecteurs de carburant comportant une pluralité d'orifices d'injection, chaque orifice d'injection ayant une entrée et une sortie, et dans lequel les injecteurs de carburant ont une ou plusieurs des caractéristiques suivantes :
(i) des orifices d'injection qui sont effilés de telle sorte que le diamètre d'entrée des orifices d'injection soit supérieur au diamètre de sortie ;
(ii) des orifices d'injection ayant un diamètre de sortie égal ou inférieur à 0,10 mm ;
(iii) des orifices d'injection dont le bord intérieur de l'entrée est arrondi ;
(iv) 6 ou plus de 6 orifices d'injection ;
(v) une température maximale de fonctionnement supérieure à 250°C.

2. Procédé suivant la revendication 1, dans lequel les injecteurs de carburant ont deux, avantageusement trois, plus avantageusement quatre, de manière hautement préférable la totalité des cinq, caractéristiques (i) à (v).

3. Procédé suivant la revendication 1 ou la revendication 2, dans lequel les injecteurs de carburant ont au moins les caractéristiques (i) et (ii), avantageusement au moins les caractéristiques (i), (ii) et (iii), plus avantageusement au moins les caractéristiques (i), (ii), (iii) et (iv).

4. Procédé suivant l'une quelconque des revendications précédentes, dans lequel au moins 25 %, avantageusement au moins 50 %, plus avantageusement au moins 80 % en poids du produit de réaction entre l'acide ou l'anhydride succinique à substituant hydrocarbyle et l'hydrazine ont un poids moléculaire qui est égal à plus de 2 fois, de préférence plus de 2,5 fois, le poids moléculaire moyen du groupe hydrocarbyle de l'acide ou de l'anhydride succinique à substituant hydrocarbyle.

5. Procédé suivant l'une quelconque des revendications précédentes, dans lequel le groupe hydrocarbyle de l'acide ou l'anhydride succinique à substituant hydrocarbyle comprend un groupe en C₈ à C₃₆, de préférence un groupe en C₈ à C₁₆ ; ou un groupe polyisobutylène ayant une moyenne en nombre du poids moléculaire de 400 à 2500, de préférence de 800 à 1200.

6. Procédé suivant l'une quelconque des revendications précédentes, dans lequel l'acide ou l'anhydride succinique à substituant hydrocarbyle et l'hydrazine sont amenés à réagir en un rapport molaire de 2:1 à 1:4, de préférence de 1:1 à 1:3.

7. Procédé suivant l'une quelconque des revendications précédentes, dans lequel le produit de réaction entre l'acide ou l'anhydride succinique à substituant hydrocarbyle et l'hydrazine est ajouté au carburant diesel en une quantité de 10 à 500 ppm en poids, de préférence de 20 à 100 ppm sur la base du poids du carburant.

8. Procédé suivant l'une quelconque des revendications précédentes, dans lequel l'entité contenant un métal comprend du zinc, du cuivre, du fer, du plomb, du cérium, un métal du Groupe I ou II, du platine ou du manganèse.

9. Procédé suivant la revendication 8, dans lequel l'entité contenant un métal comprend du zinc.

10. Procédé suivant l'une quelconque des revendications précédentes, dans lequel l'entité contenant un métal comprend un catalyseur issu du carburant.

11. Procédé suivant l'une quelconque des revendications précédentes, dans lequel la quantité de l'entité contenant un métal dans le carburant diesel, exprimée sur la base du poids total du métal dans l'entité, va de 1 à 50 ppm en poids, sur la base du poids du carburant diesel.

12. Utilisation du produit de réaction entre un acide ou anhydride succinique à substituant hydrocarbyle et de l'hydrazine comme additif dans un carburant diesel pour éliminer substantiellement les, ou réduire l'apparition de, dépôts dans les injecteurs dans un moteur diesel, le moteur étant équipé d'injecteurs de carburant ayant une ou plusieurs des caractéristiques (i) à (v) telles que définies dans la revendication 1 et fonctionnant en utilisant un carburant diesel contenant 0,1 à 50 ppm en poids, sur la base du poids du carburant diesel d'une entité contenant un métal, quantité exprimée sur la base du poids total du métal dans l'entité.
